# EUROPEAN PATENT SPECIFICATION

(11) **EP 1 850 783 B1**
(45) Date of publication and mention of the grant of the patent: **16.10.2013**
(21) Application number: 06709849.1
(22) Date of filing: 22.02.2006
(51) Int. Cl.: A61B 19/00

(54) **A PROBE FOR USE IN COMPUTER ASSISTED SURGERY**
SONDE ZUR VERWENDUNG IN COMPUTERGESTÜTZTEN OPERATIONEN
SONDE POUR CHIRURGIE ASSISTEE PAR ORDINATEUR

(30) Priority: 22.02.2005 GB 0503621
(43) Date of publication of application: 07.11.2007
(73) Proprietor: Depuy International Limited, Beeston, Leeds LS11 8DT (GB); DePuy Orthopädie GmbH, 85551 Heimstetten (DE); Biosense Webster, Inc., Diamond Bar, CA 91765 (US)
(72) Inventor: BURGER, Thorsten, 80804 München (DE); CAWTHAN, Simon, Shipley BD18 4QP (GB); LICHTENSTEIN, Yoav, 43727 Raanana (IL); NITZAN, Yaacov, Tel-Aviv (IL); REVIE, Ian, Boroughbridge YO51 9AX (GB); REZNICK, Dudi, 17096 Shimshit (IL)
(74) Representative: Belcher, Simon James
(86) International application number: PCT/GB2006/000616
(87) International publication number: WO 2006/090141

(56) References cited:
- WO-A-2004/107959
- US-A1- 2003 069 588
- US-A1- 2003 160 721
- US-A1- 2004 049 121
- US-A1- 2004 068 179
- US-A1- 2004 147 920
- US-A1- 2005 024 043
- US-A1- 2005 027 195

## Description

The present invention relates to a probe for generating position data for use in computer assisted surgery.

It has been proposed to use a computer system during surgery to assist a surgeon in navigating instruments or implants. In order for the computer system to be of use it is necessary for it to be able to build a model of particular part of the anatomy which is to be operated on. In order to generate such a model, it is known to use a position sensing probe. The probe is used to contact surfaces of the patient's tissue, anatomy or bone. Information about the location of the probe then provides information about the configuration of the surface.

Probes have been proposed which calculate the position of a tip of a probe using visible light or infrared radiation which is reflected or transmitted from markers provided on the handle of the probe. The positions of the markers on the handle of the probe are known and therefore the position and orientation of the probe handle can be calculated from the information about the positions of the markers and their relative orientation. The probe tip is at a fixed position relative to the handle, and its position can therefore be calculated from the position and orientation of the handle.

These probes have the disadvantage that the markers must remain visible to cameras in the vicinity of the operating table. In use there may be an obstruction, such as an surgeon's arm, which restricts view of the markers and preventing position information from being obtained.

US-A-2004/0068179 discusses a registration pointer for registering surfaces of bodily elements. The pointer includes a detectable device having three retro-reflective spheres. The spheres are arranged in a predetermined pattern and can be used to calculate the position of the tip of the pointer. It is possible to attach two different tips. When a tip is changed the relative position of the detectable device and the tip needs to be calibrated. After the tip has been changed, it is necessary for a user to instruct the software that the tip has been changed, or for a predetermined pattern to be traced before the pointer can be used. This can introduce user error, for example the user could enter a wrong tip configuration. It can also introduce extra complexity to the surgical procedure, for example tracing a predetermined pattern every time the pointer is used.

Systems using electromagnetic fields to obtain position have also been proposed. A position sensor in the handle of a probe senses the position using currents induced by an electromagnetic field. This overcomes the problem of requiring a line of sight to the handle. The presence of metal object in the operating theatre (notably, the operating table) can interfere with the electromagnetic field.

US-A-2003/0069588 relates to a medical instrument with a touch sensitive tip. One embodiment includes a coil in the tip that forms a "five-dimensional" magnetic tracking sensor. A combination of this coil and a coil in the instrument body together forms a "six-dimensional" magnetic tracking system. The two-part form of claim 1 is based on this document.

The present invention provides a probe in which a position sensor is mounted within the shaft or tip of the probe. By placing the position sensor in the shaft or tip it can be located close to the end of the tip and therefore remain within the usable field generated by a local position transmitter which operates with a low magnetic field.

Accordingly, the present invention provides a probe for generating position data for use in computer assisted surgery as defined in appended claim 1. The probe comprises:
a handle;
a shaft extending from the handle;
a tip attached to the shaft, the tip having a distal end for contacting an object to indicate the position of the object; and
a sensor responsive to external electromagnetic fields for generating position data with multiple degrees of freedom;
wherein the sensor is either located within the shaft towards the distal end of the shaft or located within the tip.

The tip is part of a replaceable part. At least a part of the shaft may also form part of the replaceable part.

The sensor is located within the shaft or tip to ensure that it will be within the usable field generated by a local position transmitter. The position of the distal end of the tip relative to the sensor in the shaft can be calculated because position data in multiple degrees of freedom can provide information as to both the position and the orientation of the probe.

Locating the sensor close to the tip of the probe means that, when the probe is manipulated to contact surfaces of the patient's tissue, anatomy or bone, the volume within which it moves is less than would be the case if the sensor is mounted in the handle. This can allow a signal to be generated by the sensor during movement within a low strength magnetic field such as might be provided by a local field generator. It can also help to improve the accuracy of position data which is generated using the sensor.

According to an aspect of the invention, the replaceable part comprises a passive electronic component having an electrical characteristic with a predetermined value; wherein the electrical characteristic is for indicating the geometry or configuration of the tip. The passive electronic component may be, for example, a resistor (in which case the electrical characteristic is resistance) or a capacitor (in which case the electrical characteristic is capacitance). For example, if a resistor is used, various predetermined values of the resistance of the resistor can be used to indicate the geometry or configuration of the tip. In this embodiment, information on the geometry of the tip can be communicated to the processor with low cost and complexity.

Preferably the distance from the distal end of the tip of the probe to the sensor is not more than about 150 mm, more preferably not more than about 125 mm, especially not more than about 100 mm. (This measurement is taken to the middle of the sensor.) When the tip is curved the distance is measured directly between the tip and the sensor.

Preferably the ratio of the distance from the distal end of the tip of the probe to the sensor to the distance from the distal end of the handle to the sensor is not more than about 0.5, more preferably not more than about 0.3.

Preferably the shaft has a constant cross-section at least over the portion of its length between the handle and the sensor. Preferably the transverse dimension of the shaft (which will be its diameter when the shaft has a circular cross-section) is not more than about 12 mm, more preferably not more than about 10 mm, and especially not more than about 8 mm. This allows the probe to be used in minimally invasive surgery where the shaft of the probe is required to fit into spaces which are limited as a result of the incision being small. The cross-section may be of any suitable shape, for example circular, elliptical, rectangular or square. It is however preferred to use a circular or square cross-section. In the case of a circular cross section, the transverse dimension is the diameter. In the case of a square cross-section, the transverse dimension is the side of the square.

The cross-section of the shaft can vary along its length. For example, there might be a tapered transition between the handle and the shaft, or the shaft might be tapered along a significant proportion of its length between the handle and the sensor. In this case, the shaft will be considered to be that part of the probe in which the transverse dimension is less than one or more of the limits referred to above.

In some embodiments of the invention, the interface between the handle and the shaft may not be immediately clear. In general, the shaft is considered to commence at a point where a user could not easily hold the probe, especially when the transverse dimension of the probe is less than one or more of the preferred limits specified above.

The shaft and the handle may be formed as separate pieces and bonded using a friction or interference fit, or by bonding (including welding, especially by ultrasonic welding), for example using an adhesive.

The shaft, handle and tip can be formed from a variety of materials. The tip and the shaft can be formed from a polymer such as a nylon, or a polyester, or a polyolefin, or a polyetheretherketone (PEEK), or a polyphenylene ether or a high impact polystyrene. The polymer can be reinforced by fibres, for example glass fibres or carbon fibres. The handle may also be formed from PEEK. The handle or the shaft or both can be also be formed from metal. Examples of suitable metals might include titanium and certain of its alloys. The tip and the shaft can be made from the same materials or from different materials. Factors which will affect the choice of materials might include any adverse effect on the electromagnetic field that is received by the sensor, any adverse effect on any signal that is generated by the sensor, and the mechanical properties that are required of the probe.

The shaft and handle can be formed as a single component. The shaft, handle and tip can be formed as a single component. The shaft and tip can be formed as a single component

Preferably, the sensor is contained in a bore within the shaft. This allows the sensor to be protected by the shaft itself during cleaning of the probe. It also allows the sensor to be securely positioned within the bore, for example by a friction fit or by adhesive. Preferably, the bore in the shaft is closed at the end to which the tip is attached. For example, the bore can be formed by machining from the end which is opposite to the end to which the tip is attached. Alternatively, the shaft can be formed with a bore which extends along the entire length, and be closed at the end to which the tip is attached, for example by means of a plug. The sensor should be fastened securely in the shaft against movement. It can be a tight press fit in the shaft It can be secured in place in the shaft by means of a bonding material such as an adhesive, or by locally heating the material of the shaft to cause it to soften.

Alternatively, the sensor may be contained in a bore with the tip. This allows the sensor to be easily replaced along with the tip. This is useful if the sensor has a limited operating life. For example, the sensor may be designed to be used for a single operation, or for a predetermined time span before it is replaced to ensure the accuracy of the system.

The sensor may be a Hall effect device, coils, or other antennae which can be contained in the shaft. An example of a suitable coil sensor is disclosed in US-A-2003/0120150 (Govari). The disclosed sensor includes at least one sensing coil which can generate a signal when it moves within an electromagnetic field transmitted by a local transmitter. The disclosed coil sensor is able to provide position information in multiple degrees of freedom, including up to six degrees of freedom. It can be preferred for the probe of the present invention to provide position information in six degrees of freedom, although less detailed information can be appropriate for some applications, for example at least three degrees of freedom, preferably at least four degrees of freedom, more preferably at least five degrees of freedom.

Preferably, the instrument combination of the invention is used as part of a tracking system which uses a local magnetic field generator, provided on a pad which is adapted to be affixed to a surface of the body of the patient. The pad can include a plurality of concentric orthogonal magnetic field generating coils. A driving antenna can be provided to radiate a radio frequency (RF) electromagnetic field. The pad can include a power coil which is coupled to receive the RF electromagnetic field and thereby to provide power for generating the magnetic field. Alternatively, the pad can include an internal power source to provide power for generating the magnetic field. It can however be particularly preferred for the pad to be connected by means of conductors to a source of electrical power.

A local magnetic field generator which is provided on a pad can be used in conjunction with a position sensor which is implanted in a patient's body, to provide information about the patient, in particular as to the location and orientation of the part of the body in which the sensor is implanted.

Tracking systems which comprise a pad on which a magnetic field can be generated and at least one position transducer are disclosed in US-A-2005/0245821.

The probe may include an ADC (Analogue-to-Digital Convertor) for digitising the output of the sensor. The probe may also be hard wired for output to a computer system. In that case either an analogue or a digital signal may be transmitted over the wires. If an analogue signal is used, no ADC is required.

Preferably, the probe includes a processor for processing the output of the ADC using predetermined calibration data of the position of the distal end of the tip relative to the sensor to calculate the position of the distal end of the tip, and for outputting position data of the distal end of the tip.

Calibration can be carried out at the point of manufacture. This allows the probe to be used with any system without requiring local calibration. It is possible to determine the position of the distal end of the tip, even the tip is curved away from the axis of the shaft because the position and orientation of the sensor is known to the required degrees of freedom. The processor may be implemented as a discrete component or integrated with the ADC.

Depending on the degree of miniaturisation of the processor, it may be included in the shaft with the sensor, or located in the handle.

The processor may be powered in a number of ways. For example, it may be powered by inductive coupling with an external electromagnetic field, by batteries or through a wired link if one is present.

Preferably, the processor is adapted to monitor the location of the sensor relative to the distal end of the tip and the position of the distal end of the tip. In one embodiment, the processor transmits the position data when the distal end of the tip remains in the same position and motion of the sensor relative to the tip is detected. In another embodiment the system in which the probe is used receives position data on both the sensor and the distal end of the tip and stores or analyses data when movement of the sensor relative to the tip is detected. This allows a user to indicate when the distal of the tip is in a predetermined position. For example, by simply moving the handle while maintaining the distal end of the tip in the correct position, the system can be programmed to store or analyse data relating to the position of the tip. This has the advantage that no external switch is required to inform a system that the tip is in the correct position. The construction of the probe is therefore simplified.

Optionally, the probe may further comprise a switch connected to an input of the processor. In one embodiment operation of the switch by a user causes the probe to output the current position data. This allows the position to be stored and analysed at the command of the user when the probe in the correct position.

Preferably, the probe further comprises a power source and a transmitter for transmitting the position data wirelessly. Preferably, the transmitter is contained within the handle. The power source may be a battery contained within the handle. The probe may also comprise an output socket for outputting position data over a wired connection.

Magnetic fields in the vicinity of an object being tracked can be distorted by the presence of metal or other magnetically-responsive articles. For example, in a typical surgical environment, there can be a substantial amount of conductive and permeable material, including basic and ancillary equipment (operating tables, carts, movable lamps, etc.), as well as invasive surgery apparatus (scalpels, scissors, etc). The magnetic fields produced by the field generator coils may generate eddy currents in such articles, and the eddy currents then cause a parasitic magnetic field to be radiated. Such parasitic fields and other types of distortion can lead to errors in determining the position of the object being tracked.

In order to alleviate this problem, the elements of a tracking system and other articles used in the vicinity of the tracking system are typically made of non-metallic materials when possible, or of metallic materials with low permeability and conductivity. In addition, a system controlling computer might be programmed to detect and compensate for the effect of metal objects in the vicinity of the surgical site. Suitable methods for such detection and compensation are disclosed in US-6147480, US-6373240, US-A-2004/0240240 and US-A-2005/0024043.

The tip may have a variety of configurations, depending on the particular application which it is intended for use with. The tip may be tapered towards its distal end. It may be straight or curved. The distal end of the tip may be rounded, possibly shaped as a ball, or partial sphere, with a diameter larger than the diameter of the tip immediately before the distal end.

In the event that a curved tip is used the calculation of the position of the distal end of the tip must take into account the offset from the axis of the shaft. Knowledge of the orientation of the shaft to more than one degree of freedom is then required. This is because information as to the rotational orientation of the shaft is required in addition to information as to the direction in which the shaft is pointing. In this case the offset from the sensor is expressed as a vector to convey information of the offset from the axis.

Preferably, the material of the shaft is selected so that the deflection of the tip of the shaft relative to the sensor during normal use is limited to a maximum of about 0.5 mm, more preferably about 0.1 mm, especially about 0.05 mm. By choosing the tip having an appropriate bending stiffness, the moments exerted on the tip are unlikely to give rise to significant inaccuracy during use due to deflection of the tip.

Preferably, the tip is removably attached to the shaft. During the lifetime of the probe, the tip may become damaged. However, the tip itself contains little, if any, electronic components, unlike the shaft and possibly the handle. If the tip can be replaced the probe can easily be repaired without requiring replacement of the whole probe.

In one embodiment, the tip and the shaft each comprise respective keyed engagement means for ensuring that the tip is installed in a predetermined orientation relative to the shaft. If the distal end of the tip is offset from the axis of the shaft, and the sensor is contained in the shaft, it is important the tip is replaced in the same orientation to avoid any requirement to re-calibrate the probe. The keyed engagement means, which may be a protrusion and corresponding recess, ensure that the tip can only be replaced in the correct orientation. Replacement of the tip can therefore be carried out easily by a user.

In one embodiment, at least two tips are provided for attachment to the shaft, and each of the at least two tips has a geometry such that, when installed on the shaft, the distal end of the tip is in the same relative position and orientation relative to the sensor. This allows the use of different tips, for example a tip having a pointed distal end and a tip having a hemispherical distal end, without requiring the calibration of the distal end of the tip relative to the sensor to be updated when the tip is changed. There is therefore no need for the pointer to be recalibrated every time the tip is changed as discussed in US-A-2004/0068179, reducing the possibility of error.

In a further aspect, a probe instrument kit is provided. The instrument kit comprises a probe wherein the tip is part of a replaceable part. At least two replaceable parts are provided, each replaceable part having a different geometry or configuration of the tip. The kit can provide several different tip geometries or configurations to different applications. When the replaceable part is changed, it may be possible for the geometry, configuration or calibration data to be determined automatically, for example by the embodiments described above.

In another aspect, the invention provides a system for sensing the position of a probe for use in computer assisted surgery, the system comprising:
a probe as discussed above; and
   at least one local position transmitter for generating an electromagnetic field which has a field strength of not more than about 7.5milliGauss at 200mm from the at least one position transmitter;
wherein the probe outputs position data relative to the at least one position transmitter.

Embodiments of the invention will now be described by way of example only with reference to the accompanying drawings, in which:
Figure 1 is a plan view of a probe according to an embodiment of the present invention;
Figure 2 is a cross-section along line A-A in Figure 1;
Figure 3 depicts a perspective view of a probe according to another embodiment of the present invention;
Figure 4A and 4B depict perspective views of interchangeable parts for use with the probe of Figure 3;
Figure 5 depicts the interchangeable part of Figure 4A from an alternate viewpoint, illustrating the connection features;
Figure 6 is a diagrammatic representation of the electronic system used in the embodiment of Figure 3.

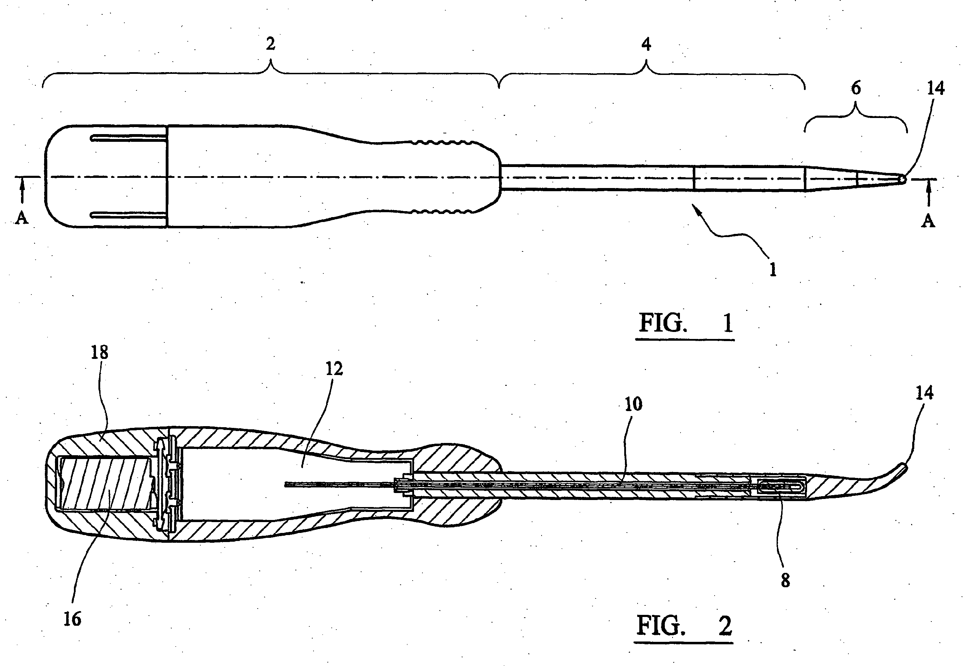

Figure 1 depicts a plan view of a probe 1 according to an embodiment of the present invention. The probe generally comprises a handle 2, a shaft 4 and a tip 6. One end of the shaft 4 is ultrasonically welded into a bore in the handle. The tip is attached to the other end of the shaft 4 by a friction fit. The handle is 125mm long and the combined length of the shaft and the tip is 110 mm. The shaft has a circular cross section with a diameter of 6.5 mm to enable use of the probe in minimally invasive surgery. The handle, shaft and tip are all constructed from highly rigid materials. In this embodiment glass- or carbon-reinforced polyetheretherketone (PEEK) is used for the shaft 4 and tip 6, with non-reinforced PEEK being used for the handle 2. This construction gives the tip a suitably high bending stiffness so that the deflection of the tip under normal use conditions is not more than about 0.05 mm. Other suitable, rigid materials may also be used.

As can be seen in the cross-section depicted in Figure 2, a position sensor 8 is fixed within a bore in the shaft 4. The position sensor comprises three coil elements for sensing a position within an external electromagnetic field. The dimensions of the position sensor are 3.5 mm wide by 3.5 mm high by 13.5 mm long, enabling it to fit within the 6.5 mm diameter shaft of this embodiment.

As can be seen in figure 2, the distal end 14 of the tip 6 is offset from the longitudinal axis of the shaft 4. The distal end 14 of the tip 6 is generally hemispherical with a diameter of 2 mm. This allows a user to access parts of the anatomy more easily with good accuracy during a minimally invasive procedure.

Referring again to Figure 2, the shaft includes a conduit 10 which runs from the position sensor 8 to a cavity 12. Connecting wires from the position sensor 8 are routed through the conduit 10 to the cavity 12 where they are connected to processing circuits (not illustrated). Also contained with the cavity 12 is a transmission circuit (not illustrated) for transmitting the position data. A power source 16, which is a disposable battery in this embodiment, is provided at the proximal end of the handle 2. The power source 16 can be replaced by a user by removing a cover 18.

The processing circuits comprise an Analogue-to-Digital convertor and a processor. The processor may be a general purpose processor, a Application Specific Integrated Circuit (ASIC), a programmable Digital Signal Processor (DSP), or any suitable alternative. The analogue signal from the elements of the position sensor 8 is converted to a digital value by the ADC and supplied to the processor which can then calculate the position of the position sensor 8 and also the distal end 14 of the tip 6.

The probe is calibrated with the precise distance between the position sensor 8 and the distal end 14 of the tip 6 before it is delivered to the user. This calibration can be carried out by placing the probe in a predetermined orientation with the tip in a predetermined position. A electromagnetic field from transmitters located at known positions is then applied. The relative position of the position sensor and the distal end of the tip can then be calculated because the only unknown is the location and orientation of the position sensor. The calibration data is stored within a non-volatile memory which can be accessed by the processor.

The probe is used in a system in combination with at least one local position transmitter in the operating area. The local position transmitter generates an electromagnetic field which induces currents in the elements of the position sensor 8. The strength of the field is relatively low, less than 7.5 milliGauss at 200mm from the local position transmitter. This is not sufficient to induce currents accurately in the position sensor 8 and limits use of the probe to situations where the position sensor is within approximately 200mm of the local position transmitter.

The currents are processed by the processor to calculate the position of the position sensor 8 relative to the local position transmitter. The processor then applies the offset determined by the calibration process to calculate the position of the distal end 14 of the tip.

During computer assisted surgery the probe 1 of this embodiment is used by a surgeon to determine the position of an anatomical feature so that the computer can create a computer model of the anatomy. The computer will provide an indication to the surgeon of which anatomical part should be located (for example by using a display screen). The surgeon then places the distal end 14 of the tip 6 of the probe 1 on that part. During this procedure the processor is monitoring the position of the distal end 14 of the tip 6 and the sensor 8. When the probe is in the correct position, the surgeon wobbles or moves the handle of the probe 1 but maintains the distal end 14 of the tip 6 in the same position. The processor detects that the sensor 8 is moving but that the distal end 14 of the tip 6 is remaining in the same position and transmits the position of the distal end 14 of the tip 6 using the transmitter.

A number of points can be identified in this way to enable a complete model to be built up. It is also possible to move the distal end 14 of the tip 6 over a bone surface to generate a model of the bone surface variations.

The tip 6 contains no electronic components and may be replaced by a user if it becomes damaged during use of the probe. However, care must be taken to ensure that the tip 6 is replaced in the same orientation otherwise the probe will need to be re-calibrated.

The specific dimensions given in the above embodiment are given by way of example in respect on one embodiment and may be varied depending on the particular surgical use which is intended of the probe. For example the shaft and tip length may be increased or decreased depending on how close to an incision the tip of the probe is required to move.

In an alternate embodiment (not illustrated) the tip and the shaft include corresponding keyed engagement means. This ensures that the tip and the shaft are always correctly aligned and enables the tip to be replaced without requiring re-calibration of the probe. The engagement means may be a shaft without rotational symmetry engaging a corresponding recess. Alternatively the engagement means could be a protrusion on the edge of shaft to engage with a recess in the side of a bore sized to receive the shaft.

In an alternate embodiment (not illustrated) a press switch is provided on the handle of the probe. The press switch is connected to an input of the processor. When a user presses the press switch, the processor transmits the current location of the distal end of the tip. Alternatively, the processor may transmit that the button is pressed so that the system knows to store or analyse the current position of the tip. A user can then easily trace a bone profile by holding the switch and moving the distal end of the tip over the bone.

In another alternate embodiment (not illustrated) a socket is provided in the handle for receiving a wired connection to the computer. The socket is connected to an output of the processor which outputs the position data of the distal end of the tip. This socket can be used in situations where a wireless link is inappropriate, or where the computer is not equipped to receive wireless data

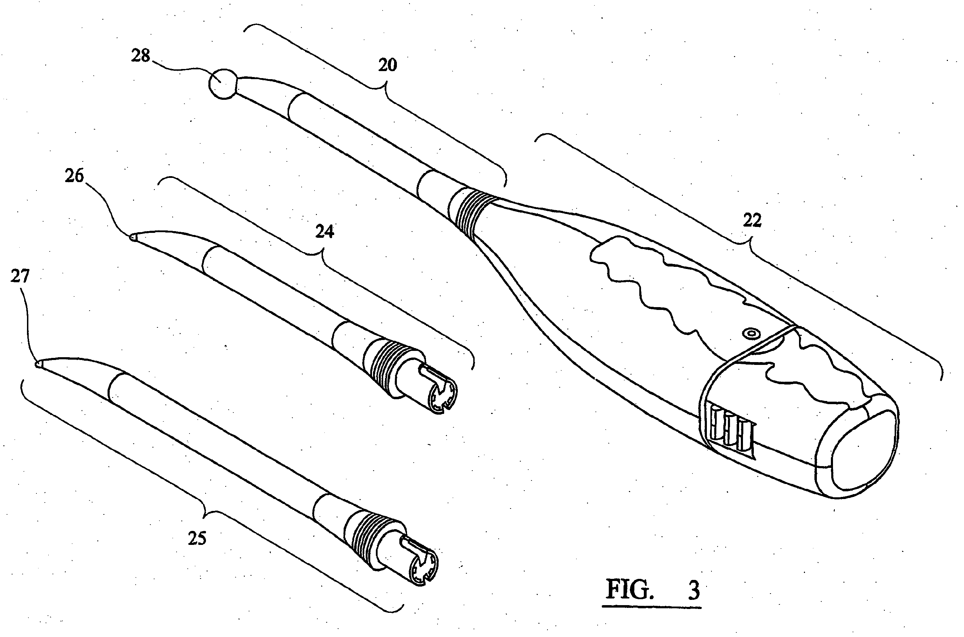

In a still further alternate embodiment depicted in Figure 3, which is the same as the first embodiment, save as described below, the tip and the shaft form a first replaceable part 20 which is removably attached to the handle 22. The position sensor is located in the replaceable part 20 and not in the handle 22. Figure 3 also depicts a second replaceable part 24 with a tip 26 and a third replaceable part 25 with a tip 27.

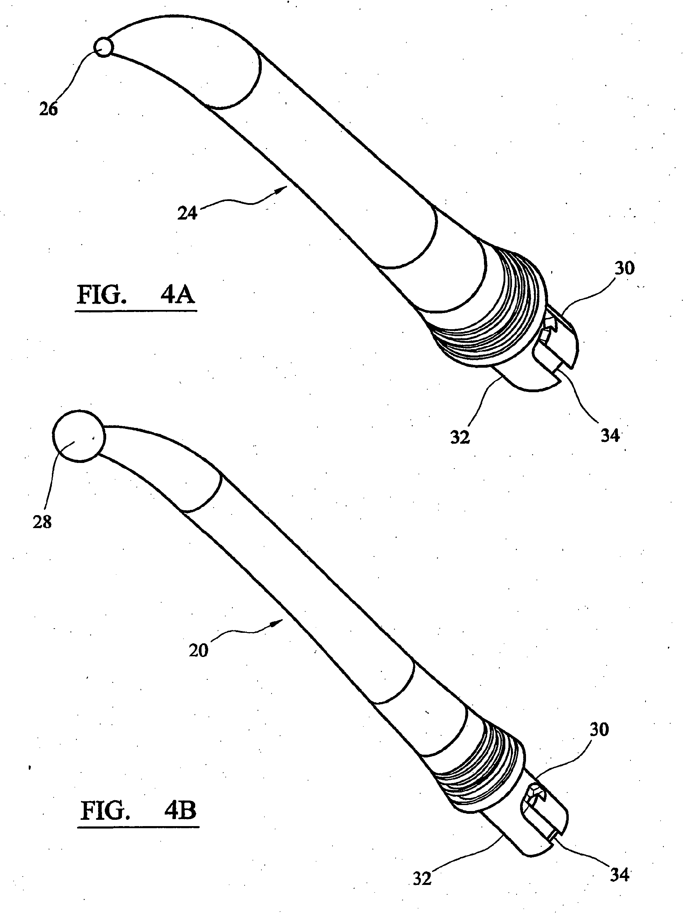

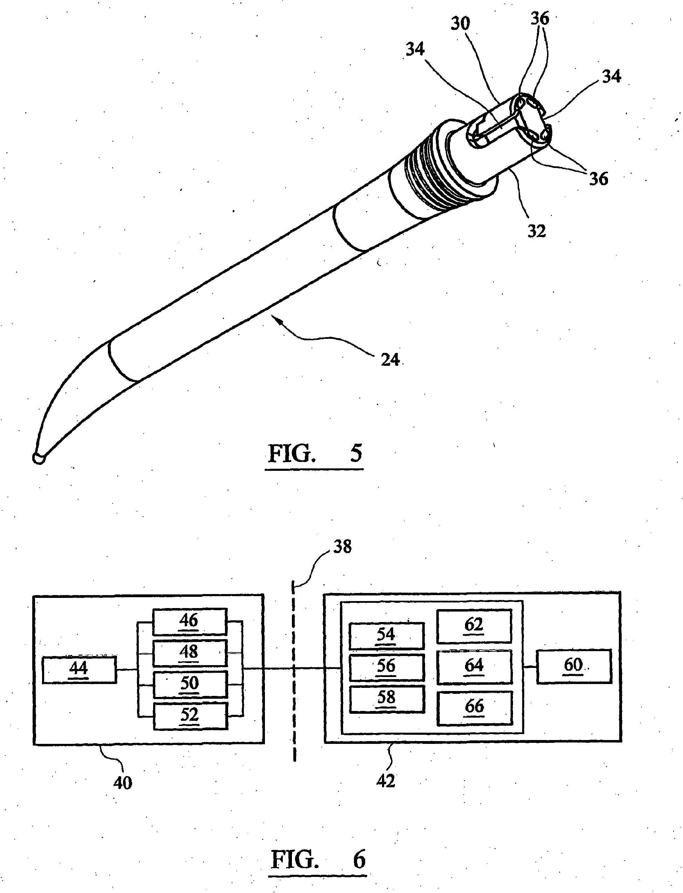

Figures 4A and 4B depict the replaceable parts when removed from the handle 22. Both replaceable parts 20 and 24 include the same physical connector 30 on their proximal end. The connector 30 comprises a generally cylindrical section 32 extending proximally. The cylindrical section has a diameter chosen to be slightly less than the diameter of a corresponding generally cylindrical recess (not shown) formed at the distal end of the handle 22. To ensure a secure connection between the replaceable part 20, 24 and the handle 22, a distally extending recess 34 forming part of a bayonet connection system is provided in the cylindrical section 32. Although Figures 4A and 4B depict one recess 34 due to the view point chosen, there are preferably two recesses provided substantially opposite each other on the cylindrical section 44, as can be seen in Figure 5. The recess 34 has dimensions chosen to receive a corresponding protrusion formed within the generally circular recess provided in the handle 22. Although a bayonet connection system has been described, other systems such as a screw fit or press fit may also be used.

As can be seen from Figure 3, the offset of the tip 26 of the second replaceable part 24 from the axis of the replaceable part is different from that of the tip 28 of the first replaceable part. Likewise the length of the third replaceable part 25 is greater than the length of the second replaceable part 24, so that the location of the tip 26, 27, 28 will depend on which replaceable is connected to the handle. The calibration of the pointer will therefore be different depending on which replaceable part is installed. The nature of the replaceable part in this embodiment is determined by an electrical connection between the handle 22 and the replaceable part 20, 24.

Figure 5 is a perspective view of replaceable part 24 showing electrical contacts or terminals 36 provided on the proximal end of the generally cylindrical section 32. Corresponding electrical contacts or terminals 36 are provided in the cylindrical recess of the handle 22, so that, when the replaceable part is attached to the handle 22, an electrical connection to components within the replaceable part is provided via terminals 36.

Figure 6 is a functional block diagram of the electronic systems used in the embodiment of Figure 3, illustrating which systems are contained in the replaceable part and the handle. the connection interface through terminals 36 is represented by the dashed line 38. Box. 40 represents the replaceable part and box 42 represents the handle. Within the replaceable part are one or more sensor coils 44 for sensing the position relative to an external electromagnetic field. The replaceable part also comprises a pre-amplifier 46, a calibration data storage circuit 48, an optional tip profile configuration data storage circuit 50 and an optional temperature sensor 52. The storage circuits may be any kind of non-volatile storage, for example ROMs or Flash memory. If the storage circuit can be updated, such as Flash memory, it can be updated during manufacture or by a user based on the individual configuration of each replaceable part. More specifically, manufacturing tolerances may mean that the position of the sensor coils 44 relative to the distal end of the tip is not always exactly the same, and in that case the calibration data can correspond exactly to the manufactured configuration.

The handle 22 receives data from the replaceable part via the connection interface 38. The handle contains a Digital Signal Processor 54, Amplifiers 56 and Analog to Digital Converters 58 for processing the data received from the replaceable part. The handle also contains the power source 60 (power is provided to the electronic systems in the replaceable part by the handle via the connection interface 38). A communication transceiver 62 for wireless and/or wired communication with a computer system or data processing equipment is also contained within the handle. In this embodiment, the handle also includes an optional acceleration sensor 64 and temperature sensor 66.

Whenever the replaceable part attached to the handle is changed, the calibration and tip configuration data is read by the handle from the replaceable part automatically. There is therefore no need for user input to inform the system of the change, reducing the possibility of user error and improving the ease of use of the system.

In an alternative embodiment (not illustrated), which is the same as the embodiment of Figure 3 save as described below, a passive component, such as a resistor or capacitor, may be provided in the tip instead of the storage circuit for calibration data. The value of the passive component, for example its resistance or capacitance, is detected through the connection interface 38. The processor in the handle, or the connected computer system can then interpret the value of the component to determine the geometry of the replaceable part and hence the calibration data to use. In this embodiment predetermined calibration data has already determined and stored for each geometry of the replaceable part.

In another alternative embodiment (not illustrated), which is the same as the embodiment of Figure 3 save as described below, a predetermined physical arrangement of electrical contacts is provided on the replaceable part. When the replaceable part is connected to the handle, the arrangement is detected, for example by determining whether particular terminals provided on the handle are electrically connected. The processor in the handle, or the connected computer system can interpret the arrangement to determine the geometry of the replaceable part and hence the calibration data to use. In this embodiment predetermined calibration data has already been determined and stored for each geometry of the replaceable part.

In other alternate embodiments, the connection between the handle and the replaceable part may be located at any point of the shaft between the tip and the shaft. At one extreme this will mean that only the tip is replaceable. At the other extreme, it will mean the entire tip and shaft is replaceable.

In further alternate embodiments the shaft may have a non-circular cross section, such as a square cross section.

The features of the various alternate embodiments may be combined with each other.

## Claims

1. A probe for generating position data for use in computer assisted surgery, which comprises:
a handle (2, 22);
a shaft (4) extending from the handle (2, 22);
a tip (26, 27, 28) attached to the shaft (4), the tip (26, 27, 28) having a distal end (14) for contacting an object to indicate the position of the object; and
a sensor (8) responsive to external electromagnetic fields for generating position data with multiple degrees of freedom;
wherein the sensor (8) is either located within the shaft (4) towards the distal end of the shaft or located within the tip (26, 27, 28); wherein the tip (26, 27, 28) is part of a replaceable part (20, 24, 25); and **characterised in that** the replaceable part comprises a passive electronic component having an electrical characteristic with a predetermined value; wherein the electrical characteristic is for indicating the geometry or configuration of the tip.

2. A probe according to claim 1, wherein the sensor (8) is contained in a bore within the shaft (4) or within the tip (6, 26, 27, 28).

3. A probe according to claim 1, wherein the transverse dimension of the shaft (4) is not more than about 12 mm.

4. A probe according to claim 1, further comprising an ADC for digitising the output of the sensor (8).

5. A probe according to claim 4, further comprising a processor for processing the output of the ADC using predetermined calibration data of the position of the distal end (14) of the tip relative to the sensor (8) to calculate the position of the distal end (14) of the tip, and for outputting position data of the distal end (14) of the tip.

6. A probe according to claim 5, wherein the processor is adapted to monitor the location of the sensor (8) relative to the distal end (14) of the tip and the position of the distal end (14) of the tip.

7. A probe according to claim 1, which includes a switch connected to an input of the processor.

8. A probe according to any one of claim 1, which includes a power source and a transmitter for transmitting the position data wirelessly.

9. A probe according to claim 8, wherein the transmitter is contained within the handle (2, 22).

10. A probe according to claim 1, which includes an output socket for outputting position data over a wired connection.

11. A probe according to claim 1, in which the tip (6) is removably attached to the shaft (4).

12. A probe according to claim 11, in which the tip (6) and the shaft (4) each comprise respective keyed engagement means for ensuring that the tip (6) is installed in a predetermined orientation relative to the shaft (4).

13. A probe according to claim 11, wherein at least two tips are provided for attachment to the shaft, and each of the at least two tips has a geometry such that, when installed on the shaft, the distal end of the tip is in the same relative position and orientation relative to the sensor.

14. A probe instrument kit comprising a probe according to claim 1, wherein at least two replaceable parts are provided, each replaceable part having a different geometry or configuration of the tip.

15. A system for sensing the position of a probe for use in computer assisted surgery, the system comprising:
a probe according to claim 1; and
at least one local position transmitter for generating an electromagnetic field which has a field strength of not more than about 7.5milliGauss at 200mm from the at least one position transmitter;
wherein the probe outputs position data relative to the at least one position transmitter.

## Patentansprüche

1. Sonde zum Erzeugen von Positionsdaten zum Verwenden bei einer computerassistierten Chirurgie, wobei die Sonde Folgendes umfasst:
einen Griff (2, 22),
einen Schaft (4), der sich von dem Griff (2, 22) erstreckt,
eine an dem Schaft (4) angebrachte Spitze (26, 27, 28), wobei die Spitze (26, 27, 28) ein distales Ende (14) zum Kontaktieren eines Objekts aufweist, um die Position des Objekts anzuzeigen, und
einen Sensor (8) zum Erzeugen von Positionsdaten mit mehreren Freiheitsgraden, der auf externe elektromagnetische Felder reagiert,
wobei sich der Sensor (8) entweder innerhalb des Schafts (4) in Richtung des distalen Endes des Schaftes befindet oder sich innerhalb der Spitze (26, 27, 28) befindet, wobei die Spitze (26, 27, 28) Teil eines austauschbaren Teils (20, 24, 25) ist, und **dadurch gekennzeichnet, dass** das austauschbare Teil ein passives elektronisches Bauteil, das eine elektrische Kenngröße mit einem vorbestimmten Wert aufweist, umfasst, wobei die elektrische Kenngröße dem Anzeigen der Geometrie oder Konfiguration der Spitze dient.

2. Sonde gemäß Anspruch 1, bei welcher der Sensor (8) in einer Bohrung innerhalb des Schaftes (4) oder innerhalb der Spitze (6, 26, 27, 28) enthalten ist.

3. Sonde gemäß Anspruch 1, bei der die Querabmessung des Schaftes (4) nicht mehr als etwa 12 mm beträgt.

4. Sonde gemäß Anspruch 1, welche weiter einen A/D-Wandler zum Digitalisieren der Ausgangsgröße des Sensors (8) umfasst.

5. Sonde gemäß Anspruch 4, welche weiter einen Prozessor zum Verarbeiten der Ausgangsgröße des A/D-Wandlers umfasst, wobei vorbestimmte Kalibrierungsdaten der Position des distalen Endes (14) der Spitze relativ zu dem Sensor (8) verwendet werden, um die Position des distalen Endes (14) der Spitze zu berechnen und um Positionsdaten des distalen Endes (14) der Spitze auszugeben.

6. Sonde gemäß Anspruch 5, bei welcher der Prozessor dazu eingerichtet ist, den Ort des Sensors (8) relativ zu dem distalen Ende (14) der Spitze und die Position des distalen Endes (14) der Spitze zu überwachen.

7. Sonde gemäß Anspruch 1, welche einen mit einem Eingang des Prozessors verbundenen Schalter enthält.

8. Sonde gemäß Anspruch 1, welche eine Leistungsquelle und einen Sender zum drahtlosen Senden der Positionsdaten enthält.

9. Sonde gemäß Anspruch 8, bei welcher der Sender innerhalb des Griffs (2, 22) enthalten ist.

10. Sonde gemäß Anspruch 1, welche eine Ausgangsbuchse zum Ausgeben von Positionsdaten über eine drahtgebundene Verbindung enthält.

11. Sonde gemäß Anspruch 1, bei welcher die Spitze (6) abnehmbar an dem Schaft (4) angebracht ist.

12. Sonde gemäß Anspruch 11, bei welcher die Spitze (6) und der Schaft (4) jeweils verzahnte Kopplungsmittel umfassen, um sicherzustellen, dass die Spitze (6) in einer vorbestimmten Orientierung relativ zu dem Schaft (4) installiert ist.

13. Sonde gemäß Anspruch 11, bei der zumindest zwei Spitzen zum Anbringen an dem Schaft vorgesehen sind, und jede der zumindest zwei Spitzen eine Geometrie aufweist, so dass, auf dem Schaft installiert, das distale Ende der Spitze sich in der gleichen relativen Position und Orientierung relativ zu dem Sensor befindet.

14. Sondeninstrumentenbausatz, der eine Sonde gemäß Anspruch 1 umfasst, wobei zumindest zwei austauschbare Teile vorgesehen sind, wobei jedes austauschbare Teil eine andere Geometrie oder Konfiguration der Spitze aufweist.

15. System zum Erfassen der Position einer Sonde zum Verwenden bei einer computerassistierten Chirurgie, wobei das System Folgendes umfasst:
eine Sonde gemäß Anspruch 1 und
zumindest einen lokalen Positionssender zum Erzeugen eines elektromagnetischen Feldes, welches 200 mm von dem zumindest einen Positionssender eine Feldstärke von nicht mehr als etwa 7,5 Milligauss aufweist,
wobei die Sonde Positionsdaten relativ zu dem zumindest einen Positionssender ausgibt.

## Revendications

1. Sonde pour produire des données de position pour l'utilisation en chirurgie assistée par ordinateur, qui comprend :
une poignée (2, 22) ;
un arbre (4) s'étendant de la poignée (2, 22) ;
une pointe (26, 27, 28) fixée à l'arbre (4), la pointe (26, 27, 28) ayant une extrémité distale (14) pour venir en contact avec un objet pour indiquer la position de l'objet ; et
un capteur (8) réagissant à des champs électromagnétiques externes pour produire des données de position avec de multiples degrés de liberté ;
où le capteur (8) est soit situé dans l'arbre (4) vers l'extrémité distale de l'arbre, soit situé dans la pointe (26, 27, 28) ; où la pointe (26, 27, 28) fait partie d'une partie remplaçable (20, 24, 25) ; et **caractérisée en ce que** la partie remplaçable comprend un composant électronique passif ayant une caractéristique électrique d'une valeur prédéterminée ; où la caractéristique électrique est pour l'indication de la géométrie ou de la configuration de la pointe.

2. Sonde selon la revendication 1, dans laquelle le capteur (8) se trouve dans un perçage dans l'arbre (4) ou dans la pointe (6, 26, 27, 28).

3. Sonde selon la revendication 1, dans laquelle la dimension transversale de l'arbre (4) n'est pas plus grande qu'environ 12 mm.

4. Sonde selon la revendication 1, comprenant en outre un ADC pour la numérisation de la sortie du capteur (8).

5. Sonde selon la revendication 4, comprenant en outre un processeur pour le traitement de la sortie de l'ADC utilisant des données de calibrage prédéterminées de la position de l'extrémité distale (14) de la pointe relativement au capteur (8), pour calculer la position de l'extrémité distale (14) de la pointe, et pour émettre les données de position de l'extrémité distale (14) de la pointe.

6. Sonde selon la revendication 5, dans laquelle le processeur est apte à surveiller l'emplacement du capteur (8) relativement à l'extrémité distale (14) de la pointe et la position de l'extrémité distale (14) de la pointe.

7. Sonde selon la revendication 1, qui comprend un commutateur connecté à une entrée du processeur.

8. Sonde selon l'une quelconque de la revendication 1, qui comprend une source de puissance et un transmetteur pour transmettre les données de position sans fil.

9. Sonde selon la revendication 8, dans laquelle le transmetteur se trouve dans la poignée (2, 22).

10. Sonde selon la revendication 1, qui inclut une prise de sortie pour émettre les données de position sur une connexion câblée.

11. Sonde selon la revendication 1, dans laquelle la pointe (6) est fixée d'une manière amovible à l'arbre (4).

12. Sonde selon la revendication 11, dans laquelle la pointe (6) et l'arbre (4) comprennent chacun des moyens d'engagement respectifs de verrouillage afin d'assurer que la pointe (6) est installée selon une orientation prédéterminée relativement à l'arbre (4).

13. Sonde selon la revendication 11, dans laquelle au moins deux pointes sont réalisées pour la fixation à l'arbre, et chacune des au moins deux pointes a une géométrie telle que, lorsqu'elle est installée sur l'arbre, l'extrémité distale de la pointe se trouve dans la même position et orientation relative par rapport au capteur.

14. Kit d'instrument à sonde comprenant une sonde selon la revendication 1, où au moins deux parties remplaçables sont réalisées, chaque partie remplaçable ayant une géométrie ou configuration différente de la pointe.

15. Système pour détecter la position d'une sonde pour utilisation en chirurgie assistée par ordinateur, le système comprenant :
une sonde selon la revendication 1 ; et
au moins un transmetteur de position locale pour produire un champ électromagnétique qui a une force de champ non supérieure à environ 7,5 milliGauss à 200mm d'au moins un transmetteur de position précité ;
où la sonde émet des données de position se rapportant à au moins un transmetteur de position précité.
